(19)

Europäisches Patentamt
European Patent Office
Office européen des brevets

(11) **EP 4 098 611 A2**

(12) **EUROPEAN PATENT APPLICATION**
published in accordance with Art. 153(4) EPC

(43) Date of publication:
**07.12.2022 Bulletin 2022/49**

(21) Application number: **21788499.8**

(22) Date of filing: **26.01.2021**

(51) International Patent Classification (IPC):
*C01B 32/15* $^{(2017.01)}$  *C09K 11/65* $^{(2006.01)}$
*B82Y 40/00* $^{(2011.01)}$  *C05G 3/00* $^{(2020.01)}$

(52) Cooperative Patent Classification (CPC):
**B82Y 30/00; B82Y 40/00; C01B 32/15; C05D 1/00; C05D 9/02; C05G 3/00; C05G 5/00; C09K 11/65**

(86) International application number:
**PCT/BR2021/050039**

(87) International publication number:
**WO 2021/207807 (21.10.2021 Gazette 2021/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.01.2020  BR 102020002172**

(71) Applicants:
• **Fundação Universidade de Brasília**
  **70904-970 Brasília (BR)**
• **Krilltech Soluções Sustentáveis em Engenharia Ltda**
  **70910-900 Brasilia (BR)**
• **Empresa Brasileira De Pesquisa Agropecuária Embrapa**
  **70770-917 Brasília (BR)**

(72) Inventors:
• **OLIVEIRA RODRIGUES, Marcelo**
  **70904-101 Brasília (BR)**
• **GOMES FARIA, Rogério**
  **73015-227 Brasília (BR)**

• **DA SILVA, Juscimar**
  **71920-010 Brasília (BR)**
• **OLIVEIRA DA SILVA, Ataílson**
  **72305-401 Brasília (BR)**
• **GALBA BUSATO, Jader**
  **70904-109 Brasília (BR)**
• **HENRIQUE SOUSA, Marcelo**
  **71936-250 Brasília (BR)**
• **EDUARDO PACHECO LIMA, Carlos**
  **71918-360 Brasília (BR)**
• **MORAES ROCHA GUEDES, Italo**
  **71915-250 Brasília (BR)**
• **BRANDÃO BRAGA, Marcos**
  **70686-170 Brasília (BR)**
• **RODRIGUES FONTENELLE, Mariana**
  **71918-360 Brasília (BR)**
• **VITÓRIA DA SILVA RODRIGUES, Carime**
  **72720-220 Brasília (BR)**

(74) Representative: **Hannke, Christian**
  **Hannke Bittner & Partner**
  **Patent- und Rechtsanwälte mbB**
  **Prüfeninger Straße 1**
  **93049 Regensburg (DE)**

(54) **PHOTOSYNTHESIS STIMULATOR BASED ON HYBRID CARBON NANOPARTICLES, RELATED PRODUCTION METHOD AND RELATED USE AS NANOBIOSTIMULANTS AND NANOFERTILIZERS IN AGRICULTURAL CROPS**

(57)    The present invention pertains to the field of agrochemicals. The invention relates to the preparation of nanocomposites based on carbon-dots with luminescent and nutrient-carrying properties that stimulate photosynthesis when applied to the leaves and/or roots of the plant, and that also stimulate physiological responses to enhance development and productivity. Such responses are typical of materials categorized is the literature as biostimulants and/or biofertilizers.

Figure 4

EP 4 098 611 A2

**Description**

**FIELD OF INVENTION**

The present patent pertains to the field of agrochemicals. The invention relates to the preparation of nanocomposites based on carbon-dots with luminescent and nutrient-carrying properties that stimulate photosynthesis when applied to the leaves and/or roots of the plant, and that also stimulate physiological responses to enhance development and productivity. Such responses are typical of materials categorized in the literature as biostimulants and/or biofertilizers.

**PRIOR STATE OF THE ART**

Continuous population growth, together with unrestrained urbanization, has been putting pressure on agriculture and other productive sectors to overcome the challenge of the rising demand for food, sustainable production in limited agricultural areas, along with a reduction in the rural labor force and adapted to climate changes (JAGGARD; QI; OBER, Philosophical Transactions of the Royal Society B: Biological Sciences, 2010**).**

According to the UN (United Nations), world population will exceed 9 billion people in 2050, and food production will have to be increased by 50-70%. Concomitantly, demand for water and energy will grow proportionally (KEATING et al. , Global Food Security, 3, 125-132, 2014).

FAO (Food and Agriculture Organization for the United Nations) has shown concern for the depletion and degradation of agricultural fields, the drastic reduction of water sources and its effects on food security and ecosystem preservation. (PRASAD; BHATTACHARYYA; NGUYEN, Frontiers in microbiology, 8, 1014, 2017). For example, demand for cereals, both for food and animal feed, can reach 3 billion tons by 2050, over the current 2.1 billion (KEATING et al., Global Food Security, 3, 125-132, 2014).

Such a pessimistic scenario urges a world effort for the development of alternative ways of meeting the increasing global demand for food, energy, and water, without adding to the pressure on natural resources (MUELLER et al., Nature, 490,254-257, out. 2012**).**

Historically speaking, agriculture is the most stable and important sector that, along several centuries, has been responsible for supplying raw materials for the food and animal feed industries. The 1960's Green Revolution helped hugely increase global food supply, as agriculture benefits from a diversity of technological innovations introduced by the biotechnology and chemical sectors. Nevertheless, the strong reliance on synthetic pesticides and fertilizers after the Green Revolution has resulted in a negative impact on the ecosystem and public health (EUROPEAN PUBLIC HEALTH ALLIANCE, Agriculture and Public Health: Impacts and pathways for better coherence 2016**).**

Abusive usage of pesticides and fertilizers contributes to the contamination of ecosystems, as well as the eutrophication of the aquatic environment and the soils (TILMAN et al., Nature, 490, 254-257, 2002*; BHAT, Asia Pacific Journal of Clinical Nutrition, 17, 91-94, 2008). Pesticide residues have been found in fresh water sources, (ALBUQUERQUE et al., Environmental science. Processes & impacts,18, 779-787, 2016) fruit, processed foods, (CABRAS, ANGIONI, J. Agric. Food Chem. 48, 967-973 2000) and even in human breast milk, (BEDI et al., Science of The Total Environment, 463-464, 720-726, 2013).

Martin et al (**2018**) observed the association between cancer and exposure to pesticides and advocate that pesticides can perform a significant role in the etiology of malignant colon cancer neoplasm, thus requiring the monitoring of the population exposure level to pesticide residues (Martin et al., Chemosphere 209, 623-631, 2018).

Besides the use of pesticides, another agricultural practice contributing to soil degradation are the irrigation systems. Almost half of the agricultural production currently comes from irrigated crops. Such a frequent practice can contribute to soil acidification, acceleration of the extraction rate of minerals in the soil or salt formation, factors that result in an eventual abandonment of such cultivation areas (PRESLEY et al., Soil Science Society of America Journal, 68, 1916-1926 2004).

Therefore, the development of sustainable agricultural techniques has been growing and, within this context, nanotechnology has emerged as a potential solution to overcome the drawbacks of conventional agriculture, since it would be able to increase productivity and, at the same time, reduce the environmental impact (BHATTACHARYYA; NGUYEN, Frontiers in microbiology, 8, 1014, 2017).

In the last few years, the gradual input of nanomaterials in agriculture for controlled-release fertilizers, pesticides and herbicides, sensors and plant growth and germination has been observed (GOGOS; KNAUER; BUCHELI, Journal of Agricultural and Food Chemistry, 60, 9781-9792 2012**;** VISHWAKARMA et al., Nanomaterials in Plants, Algae, and Microorganisms, 1, 473-500, 2017**;** MULLEN, Nature Nanotechnology, 14, 515-516 2019). Several reports show an increasing trend of scientific papers and patent applications in agricultural nanotechnology, mainly for disease management and crop protection (FRACETO et al., Frontiers in Environmental Science, 4, 20 2016). In addition, nanomaterials are already being used for plant breeding and genetic engineering purposes (AMENTA et al., Regulatory Toxicology and Pharmacology, 73, 463-476 2015).

A number of studies address the beneficial role of nanotechnology in the agricultural sector in several different ways (KUMAR et al., Journal of Environmental Science and Technology, 16, 2175-2184 2019). In a recent report, the advantages and limitations on the use of inorganic nanofertilizers in the industry, as well as the effective and efficient way of applying nanofertilizers and its consequent economic return applicability has been highlighted (RALIYA et al., Metallomics, 7, 1584-1594, 2018).

In spite of that, the use of nanotechnology in agricultural applications is still marginal compared to other industrial sectors (CHANDRIKA et al., Nanotechnology Prospects and Constraints in Agriculture, 159-186, 2018). The concrete implementation of nanotechnology in agriculture has gaps that need to be bridged so as to ensure the safety of the population in general. Other limitations of nanotechnology in agriculture and in food development are the regulation issues and the public opinion (FRACETO et al., Frontiers in Environmental Science, 4, 20 2016).

Furthermore, the vast majority of studies concerning the use of nanofertilizers has been focused on the delivery of micronutrients (zinc, copper, iron, manganese), while the intelligent delivery of macronutrients (potassium, nitrogen, sulfur, calcium, and magnesium) has been neglected (RALIYA et al., Metallomics, 7, 1584-1594, 2018).

Among the nanostructures already being used in the agribusiness sector, inorganic nanomaterials (metallic oxide and metallic nanoparticles (NP)) represent 55% of the entire application in this sector, while nanoencapsulates represent 26%, nanocomposites correspond to 7% and carbon-based nanomaterials (carbon nanotubes, black carbon and furelene) are explored in just 6% of reported technologies (PETERS et al., Trends in Food Science & Technology, 54, 155-164 2016).

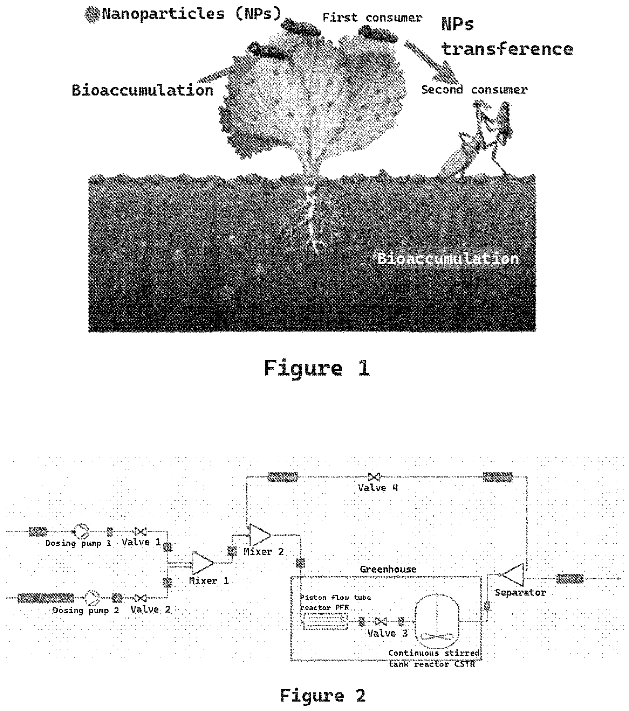

Metallic nanoparticles can also be used for the control of plant diseases and stress tolerance. Silver nanoparticles, for instance, have shown excellent results against several plant pathogens thus suggesting promising possibilities in agriculture (WORRALL et al. Agronomy, 8, 258, 2018). However, some studies show that these nanomaterials may present bioaccumulation and trophic transfer of soil granulates and nanoparticles through the food chain (**Figure 1**), (DE LA TORRE ROCHE et al., Environmental Science and Technology, 49, 11866-11874, 2015; UNRINE et al., Environmental Science and Technology, 46, 9753-9760, 2012; RALIYA et al., Metallomics, 7, 1584-1594, 2018) and, in addition, may induce change in the soil biota balance (GE; SCHIMEL; HOLDENA, Applied and Environmental Microbiology, 78, 6749-6758, 2012).

Human and environmental bioaccumulation of nanoagrochemical residues is a problem to be solved since it is bound to increase with the gradual adding up of such additives in the productive chain, and it is necessary to take into consideration all the levels in which these residues may be found and also determine the safety parameters for such inputs thus ensuring minimal environmental impact.

Within this context, the present invention outperforms all technologies using metallic nanoparticles as it is based on carbon nanoparticles, or carbon-dots, which are fully metabolized by the plant and are not transferred within the food chain, that is, there is no trophic transfer.

Moreover, metallic nanoparticles have an effect on soil microorganisms. Ge et al (**2012**) have assessed the effect of $TiO_2$ and ZnO nanoparticles in the soil bacterial community by means of DNA-based fingerprint analysis (GE; SCHIMEL; HOLDENA, Applied and Environmental Microbiology, 78, 6749-6758, 2012). Results obtained showed that these materials have induced change in the soil biota balance. A reduction in *Rhizobiales, Bradyrhizobiaceae* and *Bradyrhizobium* (nitrogen-fixed bacteria) populations and a positive impact on the *Sphingomonadaceae* and *Streptomycetaceae* populations were observed (GE; SCHIMEL; HOLDENA, Applied and Environmental Microbiology, 78, 6749-6758, 2012).

To that end, carbon nanomaterials arise as promising for the development of a sustainable agricultural alternative since they are provided with low toxicity, are biocompatible and not bioaccumulable. Carbon nanomaterials showed beneficial effects on seed germination and in the increased growth of plants besides acting as fertilizers. (ZHENG et al., ACS Omega, 2, 3958-3965, 2017).

Carbon-dots is a class of paracrystalline spheroidal nanoparticles with dimensions smaller than 10nm and composed of a highly ordered polyaromatic domain (core) enclosed by an amorphous carbon border (CHOI et al., Chemistry - An Asian Journal, 13, 586-598, 2018). Carbon-dots were first isolated in 2004 during the purification process of single-walled carbon nanotubes (XU et al. Journal of the American Chemical Society, 120, 12737-12737, 2004).

A number of Carbon-dots preparation methods are described in the prior state-of-the-art.

Liu et al (**2016**) employ the electrochemical method for the Carbon-dots synthesis by using graphite electrodes as a source of carbon (LIU et al., Analyst, 141, 2657-2664, 2016). Researchers use graphite rods as cathode and anode in an electrochemical cell with sodium hydroxide/ethanol as an electrolyte solution. The passage of current through the electrochemical circuit results in the formation of graphite rod chips thus yielding Carbon-dots with different luminescence features (varied wave lengths). As the Carbon-dots obtained by the authors are polydisperse, differences in the luminescent behavior may be attributed to mixed sizes and the distinct superficial defects. Another disadvantage to the method is the low product yield, high cost of the graphite rods and the need of a purification stage. It is important to highlight that the aspects mentioned above make the commercial use of Carbon-dots generated from electrolysis unfeasible.

The electrochemical route was also described, (RAMILA DEVI; VIGNESH KUMAR; SUNDRAMOORTHY, Journal of the Electrochemical Society, 165, G3112-G3119, 2018) proposing variations in the composition of the electrolytic solution so as to achieve other properties. However, a clear limitation in the electrochemical method is the large particle size distribution and the morphology that, at the end of the synthesis in general, requires a later separation and purification of the material obtained (RAMILA DEVI; VIGNESH KUMAR; SUNDRAMOORTHY, Journal of the Electrochemical Society, 165, G3112-G3119, 2018).

Soot generated in several reaction processes involving carbon sources, such as combustion or burning, has also been used as a precursor for the synthesis of Carbon-dots. These materials/by-products were placed into reflux systems with concentrated acids (such as nitric acid) followed by size separation of the smaller nanoparticles (LIU et al., Angewandte Chemie, 46, 6473- 6475, 2007). The strong oxidation properties of the acid are crucial for dissolution of carbon aggregates in the soot; the acid further reacts with the carbon colloids to produce oxygen- and nitrogen- rich surface defects associated with the fluorescent emission of the particles. Such soot-based or carbon-rich by-products materials, however, resulted in low yield carbon-dots and form a large quantity of toxic residues.

Hydrothermal treatment of carbon sources has become the most common procedure for Carbon-dot production. This approach enables the generation of nanoparticles from a variety of sources such as molecules and residues (DE YRO et al., AIP Conference Proceedings, 2083, 0200007, 2019).

The essence of hydrothermal synthesis of Carbon-dots is a process that starts with high temperatures inducing nucleation/condensation of carbon molecules that work as building blocks for the formation of a graphitic core. Another important feature of hydrothermal synthesis schemes is that some residues from the carbon reagents are retained upon the surface of the graphitic core, thereby providing the particles varied controllable functionalities and optical properties (WANG et al., Analytical Methods, 10, 2775-2784, 2018).

In contrast with the previously mentioned methods, the hydrothermal route displays particles with narrow size distribution and consistency in the luminescent behavior. However, the limiting factors relate to the synthesis timing, generally high, and in the low yield restrained by the use of systems controlling the inner pressure.

De Medeiros et al (**2019**) obtained Carbon-dots via microwave-based hydrothermal synthesis. Microwave irradiation replaces the heat source used in the conventional hydrothermal method. Carbon-dots generated by microwave heating emit high luminescence both in solution and in the solid phase. Synthesis time is fairly reduced, since the amount of energy released along the process is quite high and can be controlled within the equipment itself. Resulting particles are similar to those obtained via the hydrothermal method, although the yield is reduced and limited to the strength of the accessories used in the synthesis (DE MEDEIROS et al., Journal of Materials Chemistry C,7, 7175-7195, 2019). The above-mentioned aspects hinder large-scale production of Carbon-dots via hydrothermal reaction.

High-temperature carbonization for fabricating Carbon-dots has been carried out in solvents other than water. Researchers at the Max-Planck Institut for Polymerforschung showed that heat treatment of carbon sources such as citric acid dispersed in noncoordinating solvents, for instance, the organic solvents octadecene and 1-hexadecylamine, produce highly luminescent Carbon-dots (WANG et al., Chemistry of Materials, 22, 4528-4530, 2010). In return, the residues from the synthesis become a hindrance for increased production of Carbon-dots in such conditions.

Previously mentioned techniques, although effective for the production of Carbon-dots, share obstacles regarding production of a high quantity of material with uniform and selectable features. Even those that are provided with a high-yield synthesis face difficulties for reproducibility and quality of the resulting material, as in the electrochemical and hydrothermal processes. It is worth mentioning that the method proposed by the present invention allows for large-scale production of Carbon-dots and differs from the previously mentioned methods as the hydrothermal method is not used, there are no toxic reagents or residues. In the present invention, the production of Carbon-dots is carried out via continuous flow reaction in a closed circuit that ensures reproducibility and high yield. The method proposed reuses all the water and residues, and no greenhouse gases are generated.

In addition to the Carbon-dots production technique, it is important to highlight that the exploration of Carbon-dots in biological environments requires attention, as the raw material and the conditions of synthetic materials are factors influencing the surface composition, functionality, and biological activity.

A few examples illustrate that hydrothermal methods result in Carbon-dots that contain fragments of the precursor molecule on their surfaces. For example, in the document reported by Zhao et al (**2017**), Carbon-dots produced by the pyrolysis of egg yolk oil exhibit anti-hemorrhagic activity similar to the drug Hemocoagulase (Zhao, Y., Zhang, Y., Liu, X. et al. Scientific Report, 7, 4452, 2017).

In the patent application CN103980894A, Carbon-dots produced from folic acid by hydrothermal route were able to selectively recognize tumor cells by specifically interacting with plasma membrane proteins that interact with folic acid (Folate Receptors). Respective Carbon-dots exhibited all the morphological and physio-chemical features which are similar to other nano particles produced from other precursors. Nonetheless, the presence of folic acid fragments on the surface makes them unique and qualified for patent protection.

In another example, Carbon-dots produced from the pyrolysis of chicken egg membranes were able to selectively recognize DNA and RNA fragments (Pramanik et al. PCCP,20, 20476, 2018). It is worth mentioning that Carbon-dots produced by the pyrolysis of other carbon sources did not exhibit that selective capacity of recognizing nucleic acids.

In the international patent application WO/2018/160142, Carbon-dots produced from the condensation of polyoxyethylene in acid conditions resulted in a nanomaterial with antibiotic properties and were incorporated into an inert polymeric film. Once again, the morphological and physio-chemical features are similar to those of Carbon-dots produced by other carbon sources, although the surface properties of the particles resulted in a specific biological activity that enabled their protection.

Thus, even though the morphological and physio-chemical aspects suggest Carbon-dots in general are carbon spheres only, the synthetic process and the carbon source influence surface composition and biological properties of Carbon-dots.

The use of Carbon-dots in agriculture as fertilizers was proposed in the document published by Zheng et al (**2017**) that discloses the use of pollen as a precursor of nanoparticles (ZHENG et al., ACS Omega, 2, 3958-3965, 2017)). The tests were carried out in hydroponic lettuce and show that plants treated with 20 mg·L$^{-1}$ of solution grow about 50%. Results do not suggest selective interaction with proteins or enzymes responsible for plant growth. The authors simply propose the use of Carbon-dots as fertilizers.

In the study published by Li et al (**2018**), five Carbon-dots produced from graphite electrolysis exhibited unique surface composition and distinct responses in rice (LI et al., ACS, Applied Biomaterials, 1, 663-672, 2018).

Significant differences in plant growth activities between the particles were described, proving that, even though sizes and physio-chemical features are similar, each particle has unique features. Moreover, it was observed that synthesized Carbon-dots interacted with the plant genetic material, promoting the expression of growth gene Os06g32600. It was further observed that C-dot interacted with RuBisCo protein, the one responsible for the fixation of atmospheric $CO_2$ (LI et al., ACS, Applied Biomaterials, 1, 663-672, 2018).

For all of the above, the present invention outperforms the other technologies as it deals with Carbon-dots having specific characteristics that mimic responses similar to those of phytohormones that interact specifically with plasma membrane proteins and were obtained by synthetic organocatalyzed processes exclusively developed to promote accelerated plant growth and transport macro and micronutrients directly into the plant cells.

The carbon-dots described in the present patent act as biostimulants, potentiating nutrient absorption, improving water use by the plant, and enhancing the photosynthesis rate, since it keeps stomas open longer and promote accelerated growth of tested cultures.

Carbon-dots biological activity hereby proposed are unique and suggest that particles action in plant metabolism happens at a molecular level which is similar to those of phytohormones such as Auxins, Guiberlines and Cytokinins and others.

For all of the above, the development of biostimulants with mimetic activity of phytohormones that activate the plasma membrane protein H-ATPase, as disclosed in the present invention, is not foreseen in the prior state of the art.

## BRIEF DESCRIPTION OF THE FIGURES

The present invention can be more easily understood based on Figures 1 to 19, whose description follows below:

**Figure 1** illustrates the food chain eutrophication process by metallic and metallic oxide nanoparticles.

**Figure 2** shows the biostimulant production scheme from the developed method.

**Figure 3** shows the Fourier transform infrared spectrum of the Carbon-dots synthesized by the developed method.

**Figure 4** shows the Carbon-dots formation and maintenance of the superficial groups resulting from the synthesis precursors.

**Figure 5** shows the absorption spectra in the UV-Vis region and the excitation emission spectra in the interval from 360nm to 620nm of the Carbon-dots.

**Figure 6** shows the absorption spectrum of chlorophyl a and b.

**Figure 7** shows in A, the XPS spectrum and the biostimulant composition by counting the macronutrients Nitrogen, Phosphorus and Potassium (N, P, K). In B, the phosphorus high-resolution spectrum. In C, the microscopy images and the high-resolution transmission electron microscopy images (HRTEM) and, in D, the particles diameter distribution.

**Figure 8** shows the Zeta potential (z) and the hydrodynamic diameter as a function of the nutrient solution volume (mL) added to a 10 ml volume of Carbon-dots in a concentration of 50 mg/L.

**Figure 9** shows the plant distribution P1(white) and P2 (blue) in the four blocks.

**Figure 10** shows the sample distributions of group P1 treated with Carbon-dots in different concentrations after 17 days (A), 28 days (B), 42 days (C) and 56 days(D) from the beginning of the treatment.

**Figure 11** shows the sample distributions of group P2 treated with Carbon-dots in different concentrations after 17 days (A), 28 days (B), 42 days (C)and 56 days (D) from the beginning of the treatment.

**Figure 12** shows the treatment distribution in the vegetation house for the groups B1, B2, B3, and B4.

**Figure 13** shows the treatment effect with Carbon-dots, in different concentrations, on the photosynthetic rate, on the right, and on water use efficiency, on the left for blocks B1 and B2 of 'Finestra' tomatoes.

**Figure 14** shows the treatment effect with Carbon-dots, in different concentrations, on the photosynthetic rate, on the right, and on water use efficiency, on the left for blocks B3 and B4 of 'Finestra' tomatoes.

**Figure 15** shows the treatment effect with Carbon-dots, in different concentrations, on water use efficiency in hydroponic lettuce crops.

**Figure 16** shows the concentration effect of Carbon-dots in the root dry mass (RDM) of corn seedlings.

**Figure 17** illustrates the corn crop system in Leonard vases, with the application of Carbon-dots via nutrient solution.

**Figure 18** shows the concentration effect of 100 mg/L of Carbon-dots in liquid photosynthesis (A), in stomatal conductance (B), in transpiration (C) and in water use efficiency (D).

**Figure 19** shows the percentage increase or decrease of nutrients in corn plants by using the Hoagland solution with Carbon-dots.

## DETAILED DESCRIPTION OF THE INVENTION

The present invention relates to a carbonaceous photosynthesis stimulator with photoluminescence properties (Carbon-dot) to be used as biostimulants and biofertilizers as, once applied to the leaves or roots of the plant, can cause physiological responses that enhance plant growth. Said physiological responses are observed in the photosynthetic variables, in the plant root characteristics, as well as in the absorption of essential micro and macro nutrients for the development cycle.

In addition, the product of the present invention shows high solubility in water and biodegradability; is fully metabolized by the plants and, therefore, is not bioaccumulative; its use reduces the negative impact of the trophic transfer of conventional nanoparticles in the food chain; and it is not toxic. For that very reason, it reduces the environmental impact regarding soil and water contamination when compared to traditional agrichemicals, thus giving it a higher potential use as input.

Nanomaterials operate as an organic carbon source and act as micro and macronutrient carriers anchored in their structure. During the bottom-up synthesis process, nanomaterials size is controlled and, therefore, activation of the active sites in their structure is controlled. Through surface engineering expertise, these active sites are built and are prone to receive the nutrients and/or stimulants that will be delivered with greater efficiency to the most diverse types of crops due to the bioaffinity of the plants with the nanocomposite and the easiness to metabolize them.

Nanomaterials shown in this technology are obtained by the flow-through method using the combination of reactors according to **Figure 2.** For a better stowage after the synthesis process, the material is submitted to another drying operation.

One of the modalities of the present invention refers to the process to produce the Carbon-dots, which is a versatile method as it allows the use of different carbon-rich matrixes as starting material, such as glutamines, organic acids, citric acid, tartaric acid, oleic acid, malic acid, fumaric acid, isocitric acid, corn starch; it is also possible to work with co-processing by using alternative sources that are able to provide a proper reagent composition, composites with the same functional groups and short chains, preferably citric acid and tartaric acid.

The method proposed is a fast process if compared to the conventional route (hydrothermal route), since there is no need for a long stabilization time to initiate the carbonization process and particle growth as it happens in the hydrothermal route. It is also a low-cost method as it can be produced in a continuous process. Water is used as solvent and no toxic waste is generated from the synthesis as the end of the process.

The method used in the Carbon-dots synthesis is a process that involves a physiochemical transformation. The route used is considered chemically green as it uses water as solvent and no toxic residues are generated from the synthesis.

In general, said modality can be understood by the process to obtain the Carbon-dots that consists of catalyzed chemical reactions and flow-through processes.

Nanoparticles are obtained from different carbon sources, namely, citric acid, glucose, aminoacids and agricultural waste such as fruit peel, fruit pulp and animal excrement; citric acid being mainly used due to the partial neutralization acid-base reaction in which a base solution, a source of the hydroxyl functional group, is used for the formation of salt from the acids and contributes to the formation of several nucleation points by means of the electrostatic repulsion induced by ions ($OH^-$) in solution form. The base solutions used can be calcium hydroxide, sodium hydroxide, potassium hydroxide, ammonium hydroxide, aniline, metallamine, urea and thiourea, preferably ammonium hydroxide.

An acid solution, a carbon source, and a base solution, a source from the functional group, are prepared and put into the flow system.

Using a dosing pump and a valve, said solutions are dosed in a mixer and, later, redirected to a second mixing vessel to receive the recycle and the dopants (micro and macronutrients or stimulants). After passing through the mixers, the reagents are pumped into a piston flow tube reactor (PFR) provided with a valve that restrains material output and keeps a constant pressure on its inside thus avoiding vaporization of the reagents, as the reactor is heated from 100 to 250 °C, preferably 150 °C.

The PFR reactor must be provided with a reduced diameter, just a few millimeters, and be built with a material with low roughness so as to reduce pressure drop during material runoff. Another key factor in the process is to keep a regimen of turbulent hydrodynamic runoff; all of that calculated by the *Bernoulli Equation,* since this reactor must be housed inside a greenhouse to heat the mixture.

Said reactor must be heated between 100 to 250 °C, depending on the concentration of the material used. The pressure inside the reactor is controlled by the outlet valve thus preventing the solution from boiling; pressure varies from reaction to reaction depending on the density of the salts involved. Said pressure is high, thus the reactor material must withstand a pressure of up to 5 bars. The flow of the material coming out of the first reactor, the tube reactor, discharges into a continuous stirred tank reactor (CSTR), also heated and with stirring activity. The outlet flow of said reactor goes through a separation system that works by a hydrocyclone density difference or a decanter that allows removing the finished product by the overflow and returns the underflow current to the reflux.

Material reflux is required until optimal operational conditions are established. Formed product density is lower than the precursor salt solution, thus, the product can be separated by this principle, removing the supernatant and recirculating the bottom material, inserting before the reactor and adding the initial reagent current. The process up to the stabilization process in which the material stays in reflux takes from 0.5 to 2 hours, preferably 1 hour; from that period on, material density starts to modify, and the product starts forming.

The process yield is 95% (713 g after a 2 hour-cycle. This amount of material is enough to be applied to 24 hectares.

This product can be stored in liquid state, or it can go through a drying process in which the material expands its surface area in approximately 5 times its volume.

The technology for production of carbon-dots has scaling characteristics that allows it a feasible transition into an industrial scale without negative impacts in the quality of the Carbon-dots formed. In tests conducted by scaling-up 100 times the production of bench-produced C-dots, the possible production and maintenance of the same properties and qualities was shown. Under a high production demand of low environmental impact, as required by the agricultural input industry, such technology is disruptive and cohesive with the new current production demands.

Versatility is also one of the main characteristics of the developed production platform, one that allows variation in its composition and in its properties with the purpose of meeting the production chain demands.

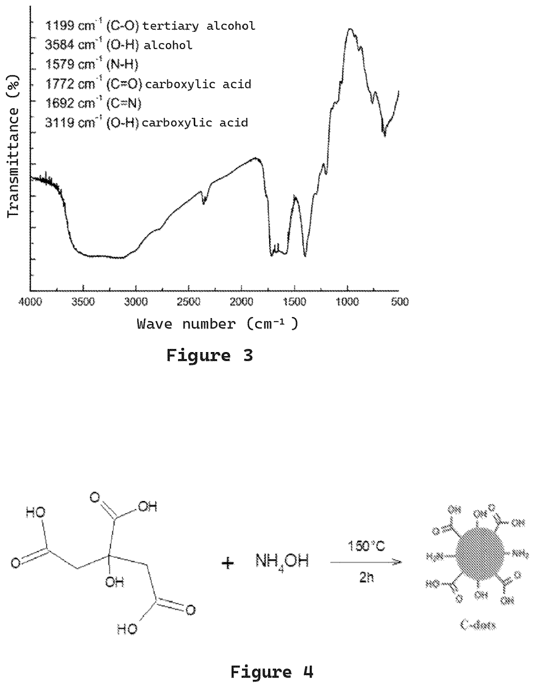

Carbon-dots produced by this process show high water solubility due to the presence of hydrophilic groups in the precursor molecules in the synthesis as can be checked by the infrared spectrum shown in **Figure 3**.

Superficial nanoparticle groups have, besides the hydrophilic character, ion complexation potential in solution **(Figure 4)**. Both the macronutrients (potassium, nitrogen, calcium, phosphorus, magnesium and sulfur), that have a high demand by the plant, and the micronutrients (manganese, boron, zinc, ferro, copper, molybdenum and chlorine), that have a lower demand, can be added to the process for preparing Carbon-dots when complexed by the superficial groups after the synthesis.

Besides that, they are biodegradable materials, which confer then a higher application potential as an agricultural input.

Therefore, another modality of the present invention refers to the use of carbon-dot based nanoformulations as powerful photosynthesis stimulators, biostimulants and organomineral biofertilizers. The technology presented in this document is fully atoxic, biocompatible, nonbioaccumuable, and sustainably produced by using the green nanotechnology principles. Test results show increased photosynthesis efficiency by about 60%, increased water use efficiency by 50%, increased root mass by 50%. Nanomaterials produced and doped with essential chemical elements simultaneously provide nutrients and improve physiological aspects of plants and reduce adverse impacts of the conventional use of fertilizers. It is important to highlight that the technology presented overcomes limitations related to economic feasibility, production scalability, environmental production and security that represent obstacles for the sustainable use of nanotechnology in agriculture.

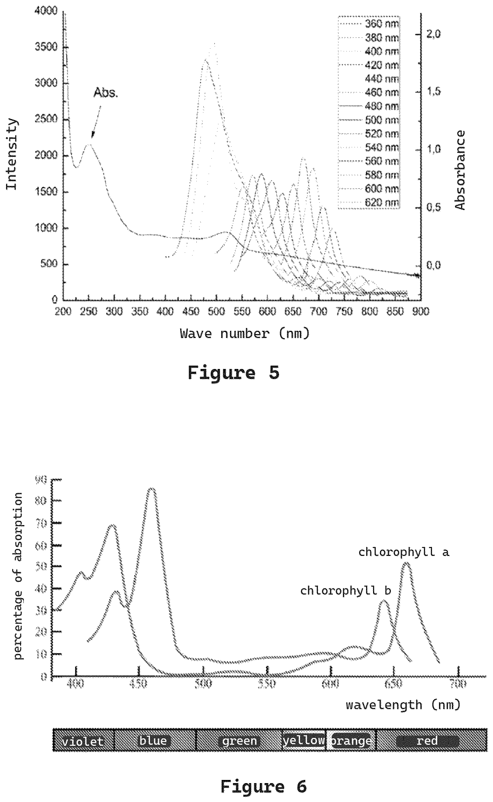

The use of Carbon-dots as photosynthetic stimulators results from its spectroscopic properties. The introduction of nitrogenated aromatic reagents, which promote the formation of C=N/C=O bonds, is responsible for the multi-state absorption and emissions in the blue/green/red areas (**Figure 5**).

Chlorophylls a and b show two absorption bands centered in the spectra areas corresponding to blue (~ 440 nm) and red (~ 650nm) but show an extremely low spectrum response for the green area (**Figure 6**). Synthesized carbon-dots show a strong absorption in the green area (532 nm) and emission centered in 645 nm. Such a spectrum adaptation results in an increased culture productivity due to a better exploitation of sunlight.

## EXAMPLES

The examples below are shown to illustrate the best embodiments of the invention. It is worth mentioning that the present invention is not limited to the examples cited and may be used in all the applications described or any other equivalent variations.

### EXAMPLE 1: Carbon-Dots Synthesis

The use in agriculture demands a higher amount than that used in laboratory. Process and production scaling was thus introduced in a pilot structure as shown in **Figure 1.** 500g of glucose and 550 mL of ammonium hydroxide were dissolved into 1000 mL of water and put in flow-through systems as show in **Figure 1.** Working temperature was set to 150°C and the final product with a 95% yield (713 g after a 2-hour cycle. This amount of material is enough to be used in 24 hectares.

### EXAMPLE 2: Hybrid Carbon-Dots Synthesis containing macronutrients

Initially, the process was not provided with a second mixer, but with the need for recycling and the demand for the input of nutrients, a second mixer was added after a first mixer to receive the acid and base currents. Thus, the recycling and the dopant dosage are calculated so as to achieve a stoichiometric ratio. That allows generating both pure C-dots and hybrid Carbon-dots with the most diverse dopants added into the second mixer.

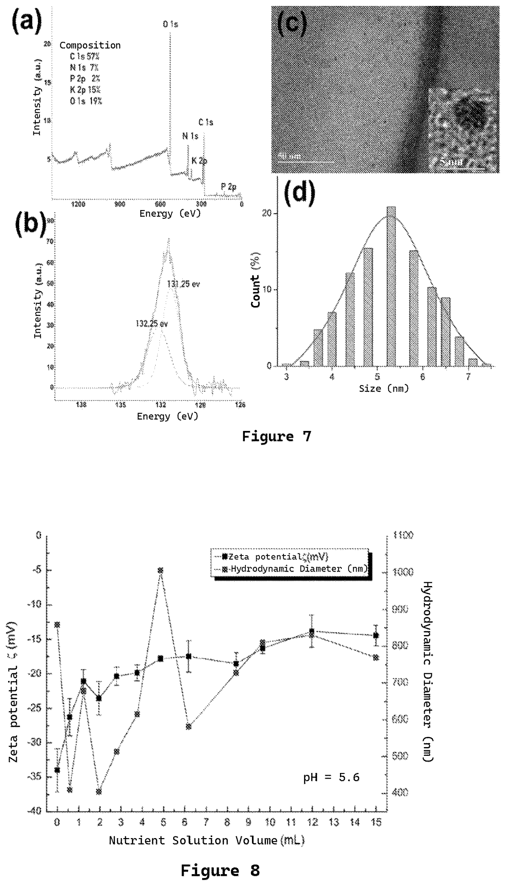

**Figure 7a** shows the XPS spectrum and sample composition containing macronutrients. High-solution XPS spectrum in **Figure 7b** shows that phosphorus (P) was incorporated into the carbon matrix. Bands centered in 131.25 and 132.25 eV correspond to groups -$C_3$-PO and -C-$PO_3$ respectively. **Figure 7c** and **d** show images of nanoparticles with spheroidal morphologies and average size of 5 nm. This result indicates phosphorous can be directly delivered to the plant cellular machinery and that the nutrient bioavailability process is similar to that of the nutrient nitrogen, which is dependent on the nanomaterial metabolization by the plant. This nanomaterial acts simultaneously as a photosynthesis stimulator, as biostimulants/biofertilizers and as a source of macronutrients (Organo-mineral fertilizers). In addition, it is fully water soluble, atoxic and, advantageously, reduces environmental impact caused by the excessive use of fertilizers in the soil. It is worth pointing out that there was no similar technology reported in the literature up to the publication of the present invention.

### EXAMPLE 3: Assessment of the nutrient carrier potential

Carbon-dots nutrient carrier potential is investigated as, in solution, carboxylic groups (COO⁻) that are a majority on the surface confer a negative charge and, therefore, a repulsion that favors the system colloidal stability. Metals categorized as micro and macro nutrients have a positive charge in solution and can be attracted by the carboxylic groups in the particles.

In order to analyze the carbon-dots surface behavior and stability when ions are added, measurements of Zeta potential (z) and hydrodynamic diameter are conducted with the increased addition of nutrient solution. A volume of 10 mL of C-dots with a concentration of 50 mg/L is titled with a standard *Hoagland* solution (1/2 strength). In **Figure 8** the behavior of the Zeta potential and the hydrodynamic diameter can be observed as a larger number of ions is added.

As expected, a decreased potential indicates that positive metallic ions bond to the surface and result in a gradual decrease in the potential. However, curve behavior tends to remain constant from a certain volume, as observed in **Figure 8.** Even with a high ionic strength, the particles show stability, confirmed by the narrow variation in the hydrodynamic diameter. These characteristics indicate C-dots can be used together with standard nutrient solutions so as to promote nutrient carriage and avoid nutrient lixiviation as it happens in the conventional agriculture. Colloidal stability also reinforces the possibility of commercialization of such technology as a photosynthesis stimulator, biostimulants/biofertilizers and liquid organomineral fertilizers.

## EXAMPLE 4: Agronomic testing

A bell pepper crop (*Capsicum annuum*) is selected to show the nanomaterial effect in the plant growth, as this crop has been on the receiving end of a lot of criticism regarding agrochemical residues in the fruits.

Carbon-dots effect on the production coefficient of the bell pepper is set up in factorial scheme 2 x 4, where two are the application uses (a single application and divided into four instalments) and four concentrations varying from 0, 25, 50 and 100 mg.L$^{-1}$ of Carbon-dots. The test is arranged in the randomized block design, with four repetitions, summing up a total of 32 experimental units.

Experimental units are plastic vessels of 5.0 dm$^3$ filled up with the mixture of inert substrate and a plant in each one. Nutrients and water are added by fertirrigation adding 400 mL of full nutrient solution four times a day.

The bell pepper seeds are sown in germination trays and, after 27 days, the emerging plantlets are transplanted to 5-liter vessels previously filled up with inert substrate that has in its composition turf, vermiculite, organic waste, agroindustrial organic waste and limestone. 32 seedlings within 19 days of transplant are selected to start carbon-dots application; size and growth criteria used is the same for all seedlings at the time of selection.

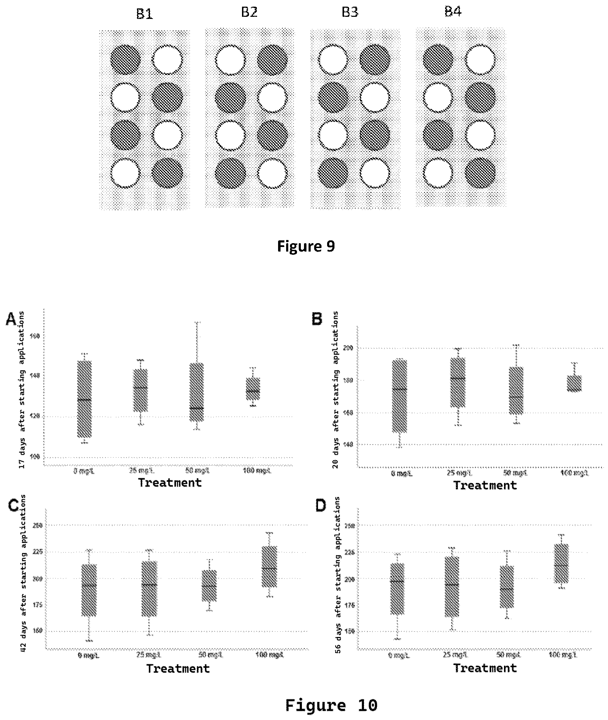

Applied carbon-dot concentrations (0 to 100 mg.L$^{-1}$) are defined from the previous tests. Maximum solution volume applied for later carbon-dot mass quantification is 200 mL for each concentration per plant. Plants are divided into two groups P1 and P2, where the plants categorized as P1 receive a single dose of solution (200 mL) and the P2 receive gradual doses (50 mL) every fifteen days until reaching maximum solution volume (200 mL). **Figure 9** represents plant division into four blocks to check treatment randomization.

The plants are placed into a glass greenhouse and evenly irrigated four times a day. Carbon-dots solutions are sprayed on the leaves so that the excess solution could freely drain into the plant substrate.

In order to measure the carbon-dots effect in the plant relative growth, a tape measure graduated in millimeters is used to measure size along the testing. In the first day, before starting the applications, initial size is measure and collected, then the first concentrations are applied. Group P1 receives a volume of 200 mL in each plant and group P2 receives the first dose of 50 mL. Size gathering process is performed every two weeks on the same day of the applications for group P2 until achieving the 200 mL volume.

**Equation 1** was used to calculate the plant relative growth, where "$h_{final}$" represents the measurement performed after the treatment application and "$h_{initial}$" the measurement performed before the treatment application.

$$\text{Equation 1: Relative growth (\%)} = \frac{(h_{final} - h_{initial})}{h_{initial}} * 100$$

Data gathered is tabulated in a spreadsheet for the application of the statistical tests. The first statistical test used is the *Shapiro-Wilk* normality test that indicated a non-normal type of distribution for the gathered data. Then, a non-parametric statistic, the *Kruskall-Wallis* tests with independent samples, is used to assess the results. (**Figures 10).** In view of that, a comparison of the averages is the most adequate manner for the identification of the statistical differences and the conclusion of the observations performed.

The effect of the concentration and the number of C-dots along the plant physiological growth is shown by means of statistical data of groups P1 and P2 separated.

From the performed statistical analysis, it can be concluded that, for group P1 (**Figure 10**), after a single application of 200 mL per plant just at the beginning of the test, there is a statistical difference between the treatment with a concentration of 100 mg·L$^{-1}$ and the control treatment of 0 mg·L$^{-1}$ after 56 days of application. There is a greater relative growth of the bell pepper when the higher concentration of carbon-dots is used (100 mg·L$^{-1}$) when compared to the other treatments, especially the control treatment, that is, without the application of the nanobiostimulant (0,0 mg·L$^{-1}$).

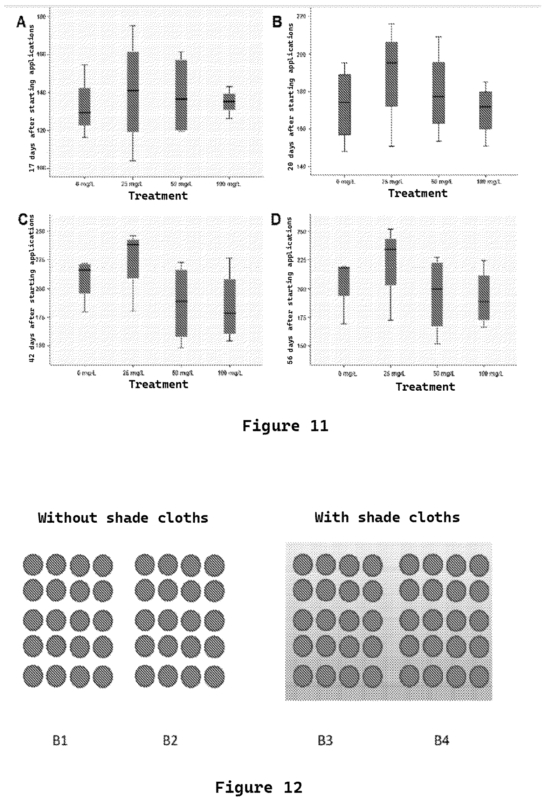

In contrast, when the result for group P2 is analyzed (**Figure 11**), where amounts of 50 mL are applied for each concentration, every two weeks until achieving a volume of 200 mL, statistical differences are observed after 42 and 56 days between the treatment with a concentration of 25 mg·L$^{-1}$ and the control treatment of 0 mg·L$^{-1}$. A smaller concentration applied in fractions contributes in a more effective manner with the relative growth when compared to the control concentration and the other treatments.

**EXAMPLE 4: Carbon-dots effect on the photosynthetic rates of 'Finestra' tomatoes**

The effect of the Carbon-dots on the photosynthetic rate is shown by using a portable photosynthesis system that uses an infrared gas analyzer. The key point of these measurements is the relationship between the $CO_2$ assimilation and the loss of water by leaf transpiration through the stomas. Specific response in the infrared both for $CO_2$ and water are used to develop sensors that read the infrared during gaseous exchanges (*Infrared Gas- exchange Analyzers - IRGAs*).

Changes in $CO_2$ and water vapor can be simultaneously monitored through the leaves, thus providing an accurate and integrated measure *in vivo* of the liquid photosynthesis and the sample transpiration when lighted by the equipment in a reading chamber that pinches the leaves without damaging them and also the mitochondrial respiration rate and the residual transpiration when the samples are in a dark environment within the reading chamber.

Carbon-dots effect on the photosynthesis is observed in 'Finestra' tomatoes, a $F_1$ hybrid developed by *Embrapa Vegetables.*

Tomato seedlings are transplanted to vases after 25 sowing days and the fruit harvest starts 60 days after the transplant.

After 48 sowing days, 5 trays with plantlets are selected to start biostimulants applications. Concentrations for this test are the following: 0 mg·L$^{-1}$ (water only); 0.5 mg·L$^{-1}$; 2.5 mg·L$^{-1}$; 5 mg·L$^{-1}$ and 10 mg·L$^{-1}$. Solutions are sprayed on the leaves so that the excess solution can drain into the substrate. The substrate used for filling the trays and the vases is made up by biostabilized pine bark, vermiculite, charcoal grinder, water, and phenolic foam.

The first dose applied on the plantlets takes place after the emergence of the true leaves still in the germination trays. A single dose of 250 mL is evenly sprayed on the trays for each treatment, and the procedure is repeated for three more consecutive days.

From the previously treated trays, 20 seedlings from each treatment are selected (0; 0.5; 2.5; 5.0; 10 mg·L$^{-1}$) to be transplanted into 15-liter vases with substrate. The seedlings are randomly distributed into four blocks thus counting four repetitions per block for each treatment and a total of 80 seedlings. Blocks B1 and B2 were exposed to light in the greenhouse while blocks B3 and B4 were covered with a shade cloth (**Figure 12**).

**Equation 1,** shown in the previous example, is used to calculate the relative growth.

After being transplanted to the vases, the seedlings go through a 15-day ripening time in the substrate, after which the applications are resumed. For the growth stage the tomato plants are in, a dose of 500 mL is applied on the leaves with the possibility of contact between the solution and the substrate. The same dose treatments (concentration) are separated and grouped for the evenly solution spraying. Then, the treatments are distributed again into the respective original benches, all care is taken so that the vases with the shade cloths are not mixed with the ones without shade cloths at the moment they are returned to the benches. Application procedure is repeated every week for four more times always in a single dose.

Data gathering is conducted after applications are stopped in the morning period and physiological studies on the mechanisms and intensities of the photosynthetic rate and transpiration along the day are carried out. Reading conditions are preserved for all treatments and after concluding the analysis, data is extracted for the application of the statistical tests in the variables of interest supplied by the equipment.

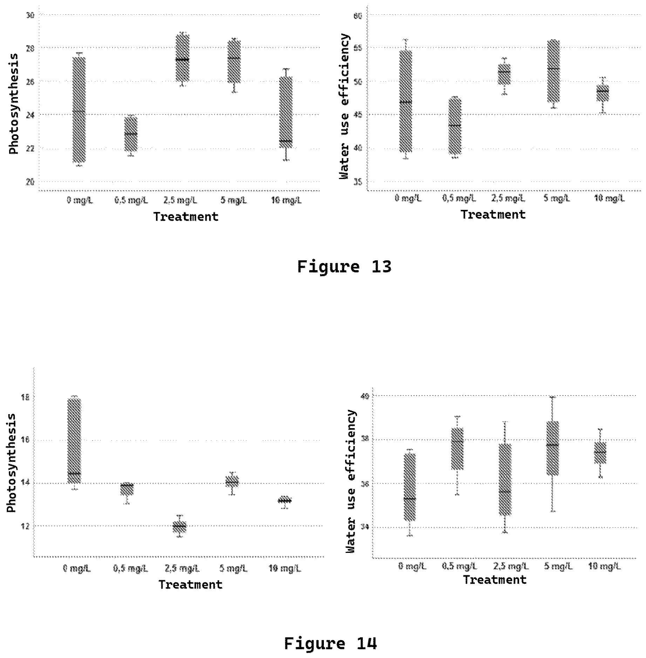

**Figures 13** and **14** show the effects of the treatments. The first statistical test employed is the *Shapiro-Wilk* normality test that indicates a non-normal distribution type for the gathered data. Then, a non-parametrical statistic, the independent sample *Kruskall-Wallis* test, is used to assess the results. In view of that, a comparison of averages becomes the most adequate manner for the identification of the statistical differences and the conclusion of the performed observations.

The combined effect of the carbon-dots concentration and the natural light in the photosynthesis of the 'Finestra' tomato plant is shown by comparing the group treated with shade cloths (B3 and B4) and the group treated without shade cloths (B1 and B2). As previously explained, carbon-dots have light absorption/luminescence and emission properties that can be explored in the light absorption process by the chlorophyll.

Data obtained for blocks B1 and B2 show that there is a tendency for an increased photosynthetic rate for the treatments with concentration of 2.5 mg·L$^{-1}$ and 5 mg·L$^{-1}$ (**Figure 13**).

Water use efficiency is a parameter not directly supplied by IRGAs but can be obtained by dividing the photosynthesis value (Pho) by the stomata conductance ($C_i$). Thus, water use efficiency in treatments B1 and B2 also tend to increase for the concentrations of 2.5 mg·L$^{-1}$ and 5 mg·L$^{-1}$ (**Figure 13**).

Data gathered for blocks B3 and B4 (**Figure 14**), both protected by the shade cloths, highlight the fact that where carbon-dots are applied, the photosynthetic rate is lower than the one in the control treatment (0 mg·L$^{-1}$). However, there is an increase in water use efficiency (**Figure 14**).

The difference between the shaded blocks and the non-shaded blocks shows that the carbon-dot luminescence behavior only takes place when the same is irradiated by an external light source.

**EXAMPLE 5: Carbon-dots effect on the photosynthetic rates of lettuce crops**

The influence of carbon-dots on the photosynthesis of lettuce (*Lactuca sativa*) cultivated in a hydroponic system is also shown. A hydroponic crop is a cultivation technique where the soil or substrate is replaced by a solution containing all the essential nutrients to plant growth.

The hydroponic system allows the seedlings to be gradually irrigated with a nutrient solution by the root. Thus, spraying is excluded and the hydroponic system itself is used as a distribution vehicle of the Carbon-dots to the plants.

Three independent benches connected to three reservoirs with independent hydroponic solutions are used. Each reservoir has a volume of 1000 liters of solution which are put into circulation in the system periodically. A Hoagland modified nutrient solution is prepared for all the reservoirs, essential for the plant growth, and the three concentrations of Carbon-dots are determined, 0 $mg \cdot L^{-1}$; 20 $mg \cdot L^{-1}$ and 50 $mg \cdot L^{-1}$. The nutrient solution itself is used as a Carbon-dot distribution vehicle.

The seedlings are obtained and go through an adaptation period in the greenhouse, where they are evenly distributed in the three benches and irrigated with the previously prepared nutrient solution. The Carbon-dots are diluted in the reservoirs and put into circulation after 21 days following the transplant of the lettuce seedlings.

Data gathering is carried out for ten lettuce seedlings in each treatment. Measurements are conducted 15 days after starting the Carbon-dots application and the reading conditions are preserved for all treatments. After the analysis is concluded, data is extracted for the application in the statistical tests as in example 4.

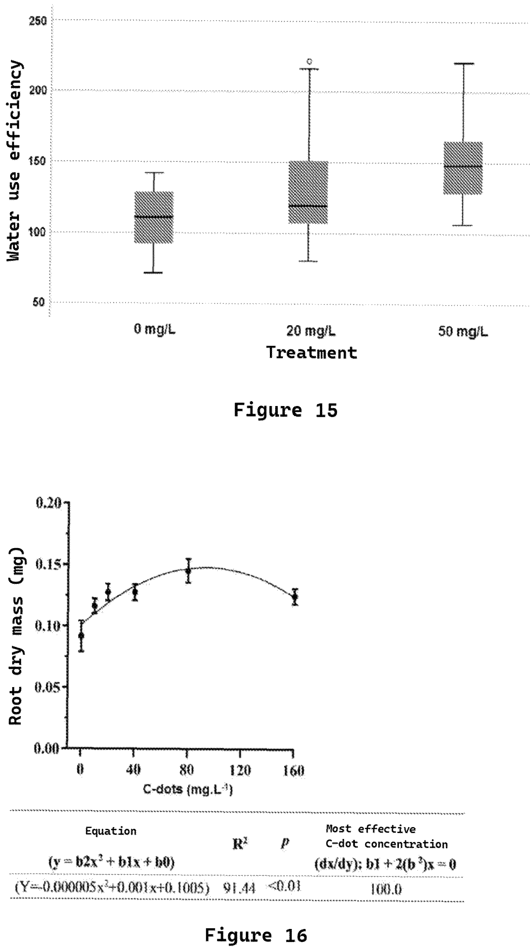

**Figure 15** shows data obtained by IRGAs. Differences can be observed only in water use efficiency for the more concentrated treatment 50 $mg \cdot L^{-1}$ and the control treatment 0 $mg \cdot L^{-1}$. These observations contribute to the fact that photosynthetic efficiency is achieved when the application takes place on the leaves but, even without promoting changes in the photosynthesis, carbon-dots can promote better use in water use during photosynthesis.

**Example 6: Effect of Carbon-dots concentration in corn seedling roots**

Corn seedlings are sonicated with a $NaClO_4$ solution at 10% for 10 minutes and rinsed with distilled water for five times for sterilization purposes. They are then placed aseptically on germination paper and germinated into a BOD chamber, in the dark, at 28°C. After two days of germination, the rooted seedlings with about 1-1,5cm are transferred to hydroponic systems in plastic vases containing *Hoagland* nutrient solution (25% strength) supplemented with 0 $mg \cdot L^{-1}$. 10 $mg \cdot L^{-1}$, 20 $mg \cdot L^{-1}$, 40 $mg \cdot L^{-1}$, 80 $mg \cdot L^{-1}$ and 160 $mg \cdot L^{-1}$ of Carbon-dots. After a 5-day growth, the plants (n = 9) are harvested and assessed for root dry weight (RDW). Results are analyzed by regression and the most efficient Carbon-dot concentration is calculated.

In order to calculate the most efficient Carbon-dot concentration, a regression analysis is carried out and the model shown here was the one that best adjusted to the data. Regression data follow a normal distribution (**Figure 16**).

A significant quadratic model is shown, with increments in low concentration and gradual inhibition the higher the Carbon-dot concentration used (**Figure 16**). The most efficient concentration is estimated in 100 $mg \cdot L^{-1}$ Carbon dots, resulting in about 75% more RDM in comparison with the control treatment.

**Example 7: Carbon-dots effect on the photosynthesis rate, gaseous exchange in the leaves and nutrient content in corn seeds**

Once the regression analysis with different doses of Carbon-dots is processed, the carbon-dots effect is shown in contrast with the control (0 $mg \cdot L^{-1}$) only and the most efficient dose observed (100 $mg \cdot L^{-1}$) as outlined in example 6.

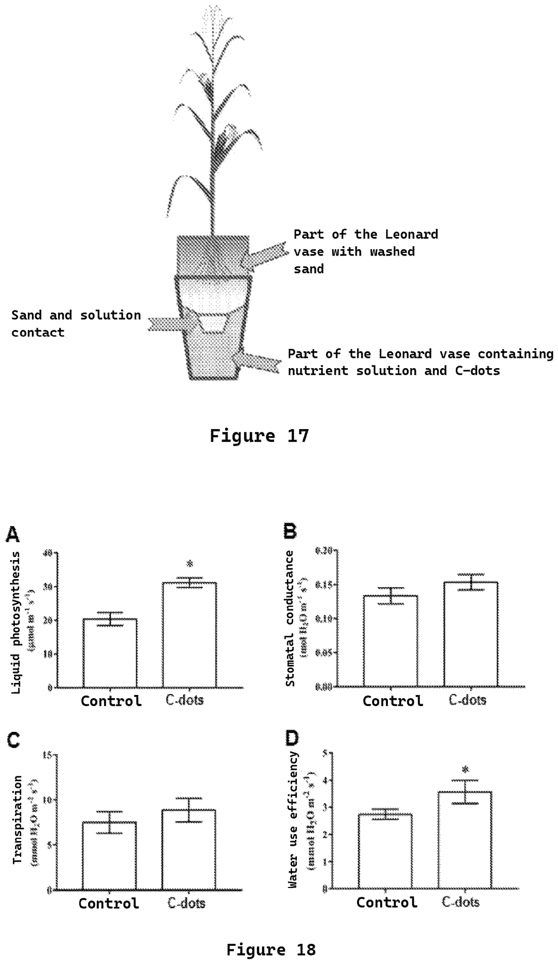

Rooted seeds with about 1- 1,5cm are transferred to Leonard vases (**Figure 17**) containing, at the top, 0.5 kg of sand (0.5-0.84 mm) sterilized with HCl solution at 10 % followed by rinsing with distilled water until obtaining an electric conductivity < 5mS·$cm^{-1}$. At the bottom of the Leonard vases, 0.5 $dm^3$ of *Hoagland* nutrient solution (25% strength) are placed, supplemented with 0 or 100 $mg \cdot L^{-1}$ Carbon-dots. Seedlings are transplanted and, every week, the solutions are replaced by newly produced solutions, but increasing concentration to 50, 75 and 100% strength.

After a 28-day growth, parameters for foliar gaseous exchange, such as liquid photosynthesis, stomatal conductance and transpiration, are measured in the plants (n = 4) by using a photosynthesis system of the *IRGAs* type, with a standard foliar chamber of 6 $cm^2$. Water use efficiency (WUE) is calculated as the ratio between liquid photosynthesis and transpiration. $CO_2$ concentration in the gaseous exchange chamber is set to 400 $\mu mol \cdot mol^{-1}$ and the measurements are carried out under photosynthetic flux density of 500 $\mu mol \cdot m^{-2} \cdot s^{-1}$. Calculations are carried out between 9 and 11 in the morning. Average temperature along the experiment is about 28°C and relative humidity is between 50 and 60%.

For the nutritional analysis, plants are harvested and heat treated at 65°C until constant weight and further ground to fine powder (2mm mesh sieve) using a knife mill. Resulting material is analyzed for Ca, Mg, S, Fe, Cu, Mn, Zn and Ni content after digestion of 0.5 g of composite with $HNO_3$ and $HClO_4$ at 180°C by atomic emission spectroscopy with a microwave plasma. Total phosphorus is estimated colorimetrically at 660 nm after digestion of composite with $H_2SO_4$ and $H_2O_2$ by the ascorbic acid/molybdenum blue acid method.

Foliar gaseous exchanges and nutrient content are analyzed by means of the F-test of variance analysis.

Addition of carbon-dots results in increased ($p < 0.05$) liquid photosynthesis (**Figure 18a**). Stomatal conductance and transpiration rate, however, are not modified (**Figures 18b** and **18c,** respectively).

Water use efficiency (WUE) has been in increasing demand in worldwide agriculture. In this respect, the use of plant stimulants should be regarded as a valuable tool since these stimulants work as root architecture modifying agents and inducers of processes that impair plant primary and secondary metabolism. WUE represents the plant capacity to optimize carbon uptake and minimize water loss, thus resulting in a competitive advantage.

Carbon-dots considerably increase WUE by 22% (**Figure 18d).** Increase in this parameter is typical of plant stimulant addition and highlights carbon-dots' ability to modulate root growth, by increasing plant capacity to absorb water from the soil.

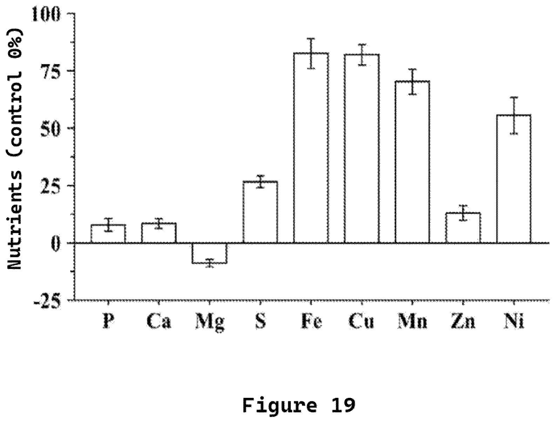

Content from all nutrients analyzed significantly changes due to the presence of Carbon-dots in the nutrient solution (**Figures 19**). Among them, reductions are observed only for Mg (8.7%). Phosphorus, Ca and Zn show the smaller increments, in the order of 7.8; 8.3 and 13.0%, respectively. Sulfur and Ni contents show intermediate increase of 26.6 and 55.5%. Largest increases are observed for Mn, Cu and Fe, whose content goes up to 70.2; 82.1; 82.6%, respectively.

Nutritional content data show that Carbon-dots work as photosynthesis stimulators and nanobiostimulants, improving absorption of some nutrients.

## Claims

1. PHOTOSYNTHESIS STIMULATOR *characterized in that* it comprises hybrid carbon nanoparticles with luminescent and nutrient-carrying properties.

2. PHOTOSYNTHESIS STIMULATOR, according to claim 1, *characterized in that* the carbon nanoparticles are doped with macronutrients, among them potassium, nitrogen, calcium, phosphorus, magnesium, and sulfur, preferably phosphorus, or micronutrients such as manganese, boron, zinc, iron, copper, molybdenum and chlorine.

3. PHOTOSYNTHESIS STIMULATOR, according to claim 1 or 2, *characterized in that* the carbon nanoparticles absorb multi-state and emissions in the blue/green/red regions.

4. PHOTOSYNTHESIS STIMULATOR, according to claim 1 to 3, *characterized in that* the carbon nanoparticles show absorption in the 532 nm wavelength region and emission centered in the 645 nm wavelength region.

5. PHOTOSYNTHESIS STIMULATOR, according to claim 1 to 4, *characterized in that* the carbon nanoparticles show spheroidal morphology with a 5 nm average size.

6. METHOD FOR PRODUCTION OF PHOTOSYNTHESIS STIMULATORS, according to one of the claims 1 to 5, *characterized in that* it is a partial acid-base neutralization reaction.

7. METHOD FOR PRODUCTION OF PHOTOSYNTHESIS STIMULATORS, according to claim 6, *characterized in that* the formation of Carbon-dots nanoparticles takes place from carbon-rich matrixes, such as glutamines, organic acids, citric acid, tartaric acid, oleic acid, malic acid, fumaric acid, isocitric acid, corn starch, preferably citric acid and tartaric acid.

8. METHOD FOR PRODUCTION OF PHOTOSYNTHESIS STIMULATORS, according to one of the claims 6 or 7, *characterized in that* the base solution used is comprised by calcium hydroxide, sodium hydroxide, potassium hydroxide, ammonium hydroxide, aniline, metallamine, urea and thiourea, preferably ammonium hydroxide and urea.

9. METHOD FOR PRODUCTION OF PHOTOSYNTHESIS STIMULATORS, according to one of the claims 6 to 8, *characterized in that* it uses water as solvent.

10. METHOD FOR PRODUCTION OF BIOPESTICIDE NANOFORMULATIONS, according to one of the claims 6 to 9, *characterized in that* the formation of carbon-dots nanoparticles takes place in a continuous flow-through system in which the acid and base solutions are injected into a mixing vessel by means of a dosing pump, being later redirected to a second mixing vessel to receive the recycle and dopants (micro and macronutrients or stimulants), then pumped to a piston-flow tubular reactor provided with a valve that restrains material output and keeps a constant pressure on the inside and a temperature between 100 and 250°C, preferably at 150°C, in which the process is kept in a regimen of turbulent hydrodynamic runoff; afterwards, the flow of material coming out of the tubular reactor is discharged into a continuous stirred tank reactor, under the same heating pattern, the output flow of said reactor passes through a separation system in which supernatant is removed and the material at the bottom is recirculated, inserting before the reactor and adding the initial reagent current; reflux process is resumed until the product is formed.

11. USE OF PHOTOSYNTHESIS STIMULATOR, according to the definition in one of the claims 1 to 5, **characterized in that** it is used as a biostimulant.

12. PHOTOSYNTHESIS STIMULATOR, according to the definition in one of the claims 1 to 5, **characterized in that** it is used as a macronutrient and micronutrient carrier.

**Figure 1**

**Figure 2**

1199 cm⁻¹ (C-O) tertiary alcohol
3584 cm⁻¹ (O-H) alcohol
1579 cm⁻¹ (N-H)
1772 cm⁻¹ (C=O) carboxylic acid
1692 cm⁻¹ (C=N)
3119 cm⁻¹ (O-H) carboxylic acid

**Figure 3**

**Figure 4**

Figure 5

Figure 6

**Figure 7**

**Figure 8**

Figure 9

Figure 10

Figure 11

**Without shade cloths**     **With shade cloths**

B1          B2          B3          B4

Figure 12

Figure 13

Figure 14

Figure 15

| Equation (y = b2x² + b1x + b0) | $R^2$ | $p$ | Most effective C-dot concentration (dx/dy): b1 + 2(b²)x = 0 |
|---|---|---|---|
| (Y=-0.000005x²+0.001x+0.1005) | 91.44 | <0.01 | 100.0 |

Figure 16

Part of the Leonard vase with washed sand

Sand and solution contact

Part of the Leonard vase containing nutrient solution and C-dots

Figure 17

Figure 18

Figure 19

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 103980894 A

- WO 2018160142 A

**Non-patent literature cited in the description**

- **JAGGARD ; QI ; OBER.** *Philosophical Transactions of the Royal Society B: Biological Sciences,* 2010
- **KEATING et al.** *Global Food Security,* 2014, vol. 3, 125-132
- **PRASAD ; BHATTACHARYYA ; NGUYEN.** *Frontiers in microbiology,* 2017, vol. 8, 1014
- **KEATING et al.** *Global Food Security,* 2014, vol. 3, 125-132
- **MUELLER et al.** *Nature,* 2012, vol. 490, 254-257
- Agriculture and Public Health: Impacts and pathways for better coherence. *EUROPEAN PUBLIC HEALTH ALLIANCE,* 2016
- **TILMAN et al.** *Nature,* 2002, vol. 490, 254-257
- **BHAT.** *Asia Pacific Journal of Clinical Nutrition,* 2008, vol. 17, 91-94
- **ALBUQUERQUE et al.** Environmental science. *Processes & impacts,* 2016, vol. 18, 779-787
- **CABRAS, ANGIONI.** *J. Agric. Food Chem.,* 2000, vol. 48, 967-973
- **BEDI et al.** *Science of The Total Environment,* 2013, vol. 463-464, 720-726
- **MARTIN et al.** *Chemosphere,* 2018, vol. 209, 623-631
- **PRESLEY et al.** *Soil Science Society of America Journal,* 2004, vol. 68, 1916-1926
- **BHATTACHARYYA ; NGUYEN.** *Frontiers in microbiology,* 2017, vol. 8, 1014
- **GOGOS ; KNAUER ; BUCHELI.** *Journal of Agricultural and Food Chemistry,* 2012, vol. 60, 9781-9792
- **VISHWAKARMA et al.** *Nanomaterials in Plants, Algae, and Microorganisms,* 2017, vol. 1, 473-500
- **MULLEN.** *Nature Nanotechnology,* 2019, vol. 14, 515-516
- **FRACETO et al.** *Frontiers in Environmental Science,* 2016, vol. 4, 20
- **AMENTA et al.** *Regulatory Toxicology and Pharmacology,* 2015, vol. 73, 463-476

- **KUMAR et al.** *Journal of Environmental Science and Technology,* 2019, vol. 16, 2175-2184
- **RALIYA et al.** *Metallomics,* 2018, vol. 7, 1584-1594
- **CHANDRIKA et al.** *Nanotechnology Prospects and Constraints in Agriculture,* 2018, 159-186
- **PETERS et al.** *Trends in Food Science & Technology,* 2016, vol. 54, 155-164
- **WORRALL et al.** *Agronomy,* 2018, vol. 8, 258
- **DE LA TORRE ROCHE et al.** *Environmental Science and Technology,* 2015, vol. 49, 11866-11874
- **UNRINE et al.** *Environmental Science and Technology,* 2012, vol. 46, 9753-9760
- **GE ; SCHIMEL ; HOLDENA.** *Applied and Environmental Microbiology,* 2012, vol. 78, 6749-6758
- **ZHENG et al.** *ACS Omega,* 2017, vol. 2, 3958-3965
- **CHOI et al.** *Chemistry - An Asian Journal,* 2018, vol. 13, 586-598
- **XU et al.** *Journal of the American Chemical Society,* 2004, vol. 120, 12737-12737
- **LIU et al.** *Analyst,* 2016, vol. 141, 2657-2664
- **RAMILA DEVI ; VIGNESH KUMAR ; SUNDRAMOORTHY.** *Journal of the Electrochemical Society,* 2018, vol. 165, G3112-G3119
- **LIU et al.** *Angewandte Chemie,* 2007, vol. 46, 6473-6475
- **DE YRO et al.** *AIP Conference Proceedings,* 2019, vol. 2083, 0200007
- **WANG et al.** *Analytical Methods,* 2018, vol. 10, 2775-2784
- **DE MEDEIROS et al.** *Journal of Materials Chemistry,* 2019, vol. 7, 7175-7195
- **WANG et al.** *Chemistry of Materials,* 2010, vol. 22, 4528-4530
- **ZHAO, Y. ; ZHANG, Y. ; LIU, X. et al.** *Scientific Report,* 2017, vol. 7, 4452
- **PRAMANIK et al.** *PCCP,* 2018, vol. 20, 20476
- **LI et al.** *ACS, Applied Biomaterials,* 2018, vol. 1, 663-672